(19) Europäisches Patentamt
European Patent Office
Office européen des brevets

(11) **EP 2 495 554 A1**

(12) **EUROPEAN PATENT APPLICATION**
published in accordance with Art. 153(4) EPC

(43) Date of publication:
**05.09.2012 Bulletin 2012/36**

(51) Int Cl.:
*G01N 23/04* (2006.01)   *A61B 6/03* (2006.01)

(21) Application number: **10826875.6**

(22) Date of filing: **29.10.2010**

(86) International application number:
**PCT/JP2010/069350**

(87) International publication number:
**WO 2011/052745 (05.05.2011 Gazette 2011/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2009 JP 2009250690**

(71) Applicants:
• **Tokyo University Of Science Educational Foundation Administrative Organization Shinjuku-ku Tokyo 162-8601 (JP)**
• **Yamagata University Yamagata-shi, Yamagata 990-8560 (JP)**

(72) Inventors:
• **ANDO, Masami Tokyo 162-8601 (JP)**
• **SUNAGUCHI, Naoki Yonezawa-shi Yamagata 992-8510 (JP)**
• **SHIMAO, Daisuke Inashiki-gun Ibaraki 300-0394 (JP)**
• **YUASA, Tetsuya Yonezawa-shi Yamagata 992-8510 (JP)**

(74) Representative: **Ström & Gulliksson AB Studentgatan 1 P.O. Box 4188 203 13 Malmö (SE)**

(54) **IMAGE SYNTHESIZING DEVICE AND IMAGE SYNTHESIZING METHOD**

(57)   Provided are an image synthesizing device and an image synthesizing method wherein a subject is less affected by beam absorption. A diffraction beam intensity and a front diffraction beam intensity actually detected are included in the influence of absorption on a subject (Sa). Yet, assuming that said beams have the attenuation rate which is caused by said beams being absorbed when passing through a subject, the angle of refraction $\theta_0$ of the beam when passing through the subject (Sa) is obtained using the diffraction beam intensity and the front diffraction beam intensity which are not affected by attenuation, and which are represented by the aforementioned attenuation rate, front diffraction beam intensity, and diffraction beam intensity; a synthesized image of the subject (Sa) is obtained by means of said angle of refraction $\theta_0$.

FIG. 1

EP 2 495 554 A1

**Description**

TECHNICAL FIELD

The present invention relates to an image synthesis apparatus and an image synthesis method for obtaining a synthesized image of a subject by applying a beam to the subject and by using the beam refracted by the subject.

BACKGROUND ART

Image diagnosis by means of X-rays has greatly contributed to the advancement of the medical science since the discovery of X-rays by Roentgen in 1895. In particular, X-ray CT developed by Hounsfield, et al. is an epoch-making invention in that it made a quantum leap in diagnosis accuracy. X-ray CT technology has developed steadily since its emergence and also continues developing presently. Four-dimensional CT capable of providing three-dimensional tomographic information in real time as well as submillimeter spatial resolution is also about to reach a stage of practical use.

However, there is a limit to the conventional X-ray image taking techniques according to the principle. The X-ray image taking techniques presently used in clinical practices utilize absorption characteristics specific to physical matters to produce a contrast. X-ray absorption is useful in producing a sufficient contrast with respect to high-atomic-number elements such as Ca constituting bones but is significantly weak with respect to low-atomic-number elements such as C, H and O constituting soft part tissues. Therefore, a conventional image taking based on absorption contrast does not produce a sufficient shading effect in taking an image of a soft part tissue. For this reason, it has been pointed out that a considerably large number of lesions have not been noticed in such mammographic images. In contrast, a refraction phenomenon provides a sensitivity of 1000 times higher than absorption in a hard X-ray region, which is expected to provide a high contrast even with respect to a soft part tissue.

On the other hand, with the development of synchrotron radiation sources, use of high-quality parallel monochromatic X-rays has been made easier. It has, therefore, become possible to obtain images with significantly high definition in comparison with images taken by using a conventional X-ray tube as a light source. Efforts are under way to use the excellent characteristics of synchrotron radiation X-rays and to develop and study image taking techniques for producing a contrast by means of an X-ray refraction phenomenon.

Among such techniques, an X-ray diffraction-enhanced imaging (DEI) method and an X-ray dark-field imaging (DFI) method have enabled taking an image even from a subject constituted by low-atomic-number elements with a contrast much higher than an absorption contrast by discriminating X-rays refracted by a subject with an analyzer made from a Si monocrystal thin plate. Studies are being energetically made for clinical applications such as applications to mammography and observation of a rheumatic bone lesion (see, for example, Patent Literature 1).

Patent Literature 1: International Publication No. WO2006/090925

DISCLOSURE OF THE INVENTION

Problems to be Solved by the Invention

However, there is a problem that images obtained by the conventional DEI and DFI methods are influenced by absorption in subjects. The intensity of X-rays obtained from the subject is thereby reduced, so that the true refractive index cannot be obtained and quantitative diagnosis is made difficult. As a result, correct refraction information on the projection cannot be obtained and a refractive index distribution CT image cannot be obtained.

Conventionally, in the DEI method, an image is taken two times while an angular analyzer is held at two angles obtained from a rocking curve (reflection points on the rocking curve at which the maximum of angle information can be extracted from contrast), thereby removing the influence of this absorption. This technique, however, increases the exposure of the subject to X-rays. Also, if the subject moves when image taking is performed by changing the angle of the angular analyzer, a correct synthesized image cannot be obtained. In the DFI method, the influence of the background is small and, therefore, taking of images at two angles obtained from a rocking curve in the conventional way is not performed and no measures are taken against absorption.

A task of the present invention is to provide an image synthesis apparatus and an image synthesis method in which the influence of a move of a subject is small and the influence of absorption of a beam in the subject is small, while an increase in the exposure of the subject to the beam is avoided.

Means for Solving the Problems

(1) To achieve the above-described task, according to the present invention, there is provided an image synthesis apparatus including an angular analyzer that separates a parallel beam applied to a subject into a forward diffracted beam and a diffracted beam, a detection device that detects a first forward diffracted beam intensity, which is the intensity of the forward diffracted beam separated by the angular analyzer, and a first diffracted beam intensity, which is the intensity of the diffracted beam, and a computation device that obtains an angle of refraction of the beams by the subject from a forward diffraction curve representing the relationship between the intensity of the forward diffracted beam and the refraction angle and a diffraction curve representing the relationship between the intensity of the diffracted beam and the refraction angle, the curves representing characteristics of the angular analyzer, by assuming that an attenuation rate at the first forward diffracted beam intensity and an attenuation rate at the first diffracted beam intensity at the time of passage through the subject are equal to each other, and by using a second forward diffracted beam intensity and a second diffracted beam intensity from which the influence of attenuation is removed, the second forward diffracted beam intensity and the second diffracted beam intensity being obtained by correcting the first forward diffracted beam intensity and the first diffracted beam intensity by the attenuation rate, the computation device synthesizing an image of the subject from the refraction angle.

(2) To achieve the above-described object, according to the present invention, there is also provided an image synthesis method including separating a parallel beam applied to a subject into a forward diffracted beam and a diffracted beam by means of an angular analyzer, detecting a first forward diffracted beam intensity, which is the intensity of the forward diffracted beam separated by means of the angular analyzer, and a first diffracted beam intensity, which is the intensity of the diffracted beam, and obtaining an angle of refraction of the beams by the subject from a forward diffraction curve representing the relationship between the intensity of the forward diffracted beam and the refraction angle and a diffraction curve representing the relationship between the intensity of the diffracted beam and the refraction angle, the curves representing characteristics of the angular analyzer, by assuming that an attenuation rate at the first forward diffracted beam intensity and an attenuation rate at the first diffracted beam intensity at the time of passage through the subject are equal to each other, and by using a second forward diffracted beam intensity and a second diffracted beam intensity from which the influence of attenuation is removed, the second forward diffracted beam intensity and the second diffracted beam intensity being obtained by correcting the first forward diffracted beam intensity and the first diffracted beam intensity by the attenuation rate, and synthesizing an image of the subject from the refraction angle.

The first forward diffracted beam intensity and the first diffracted beam intensity actually detected include the influence of absorption in the subject. According to the present invention, however, the rates of attenuation of the beams due to absorption at the time of passage through the subject at the first forward diffracted beam intensity and the first diffracted beam intensity are assumed to be equal to each other; and an angle of refraction of the beams at the time of passage through the subject is obtained from a second forward diffracted beam intensity and a second diffracted beam intensity, which do not include the influence of attenuation, expressed by this attenuation rate, the forward diffracted beam intensity and the diffracted beam intensity; and a synthesized image of the subject is obtained from the refraction angle. Thus, an image not including the influence of absorption of the beams in the subject can be obtained. In the conventional DEI, the influence of absorption is removed by applying the beam to the subject two times. In contrast, according to the present invention, the influence of absorption can be removed by one application. Therefore, an image not including the influence of absorption can be obtained with an total exposure equal to 1/2 of the total exposure in the conventional art. Further, in the case of the conventional art applying the beam to the subject two times to remove the influence of absorption, the image is made unsharp if the subject moves. In the case of the present invention, a synthesized image can be obtained by applying the rays one time and, therefore, the image is not made unsharp even if the subject moves.

(3) The above-described computation device may approximate the forward diffraction curve with an n-degree polynomial to obtain a new forward diffraction curve, approximate the diffraction curve with an n-degree polynomial to obtain a new diffraction curve, thereafter if it is assumed that the X-rays that pass through the subject in the forward refraction and the refraction receive equal amounts of attenuation from the subject, an n-degree equation to be satisfied by the corresponding two observed intensities can be derives. By solving the equation, the refraction angle can be obtained.

(4) As above-described angular analyzer, a transition-type angular analyzer may be used. In such a case, a synthesized image can be obtained through a dark field. A reflection-type angular analyzer may alternatively be used as the above-described angular analyzer.

(5) The beam is, for example, electromagnetic waves or neutron rays.

(6) The above-described detection device may include a first detection device that detects the forward diffracted beam separated by the angular analyzer, and a second detection device that detects the diffracted beam. If two detection devices are provided, the corresponding beams can be detected separately from each other.

Effects of the Invention

According to the present invention, an image synthesis apparatus and an image synthesis method in which the influence of absorption of a beam in a subject is small can be provided.

BRIEF DESCRIPTION OF THE DRAWINGS

Figure 1 is a diagram schematically showing an image synthesis apparatus in a first embodiment of the present invention.
Figure 2 is a graph showing intensity curves with respect to a first crystal and a second crystal.
Figure 3 is a graph showing a diffraction intensity curve of diffraction of X-rays at a Bragg case (first crystal).
Figure 4 is a graph showing an intensity curve as a result of convolution in X-ray form of the intensity curves with respect to the first crystal and the second crystal.
Figure 5A shows a forward diffraction intensity curve representing a forward diffraction intensity characteristic of an angular analyzer.
Figure 5B shows a diffraction curve representing a diffraction intensity characteristic.
Figure 6 is a diagram showing an in-specimen route L for incident X-rays.
Figure 7 is a diagram showing a basic CT construction.
Figure 8A-8D show images as a result of CT reconstruction from a projected image obtained by simulation.
Figure 9 is a graph of a refractive index real part for one vertical line in a CT image.
Figure 10 is a photograph actually taken from a subject by using the technique in the first embodiment.
Figure 11 is a diagram schematically showing an image synthesis apparatus in a second embodiment.
Figure 12 is a diagram schematically showing an image synthesis apparatus in a third embodiment.
Figure 13 is a diagram schematically showing a modified example of the third embodiment.

EXPLANATION OF REFERENCE NUMERALS

| | |
|---|---|
| 1, 100 | Image synthesis apparatus |
| 11, 111 | First crystal (parallelizing means) |
| 12, 112 | Second crystal (angular analyzer) |
| 13, 14, 113, 114, 130 | CCD camera |
| 20, 120 | Computation device |
| A | Forward diffracted wave |
| B | Diffracted wave |
| Sa | Subject |

PREFERRED MODE FOR CARRYING OUT THE INVENTION

(First Embodiment)

With respect to a case where a parallel beam applied to a subject is X-rays, for example, a first embodiment of the present invention using an X-ray dark-field imaging (DFI) method will be described. The parallel beam is not limited to X-rays as long as the parallel beam has characteristics of a wave and can be diffracted. The parallel beam may be any other electromagnetic waves such as visible rays, or neutron rays.

(Description of Apparatus)

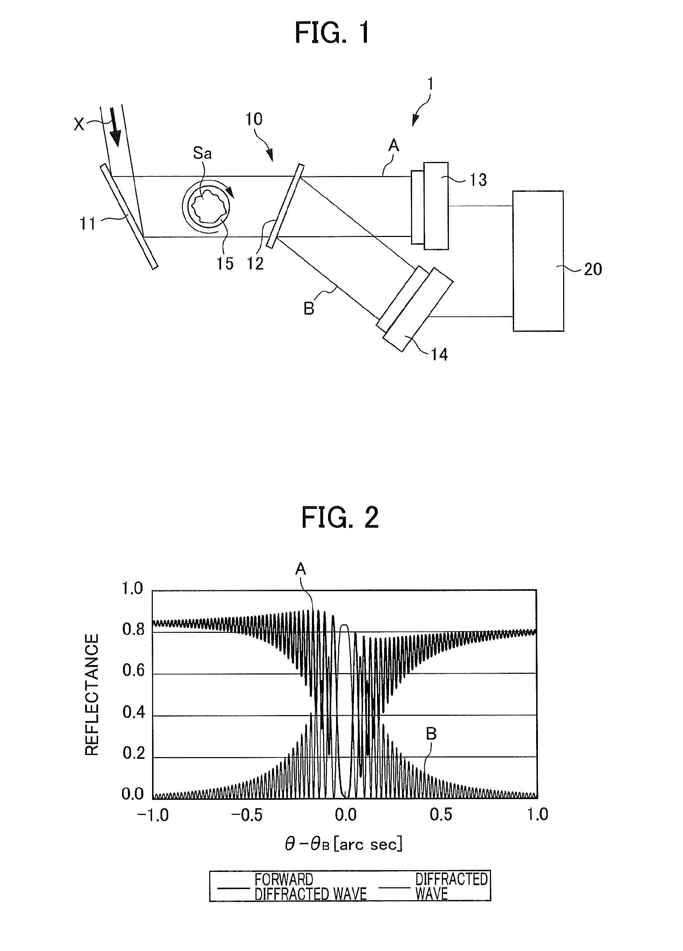

Figure 1 is a diagram schematically showing an image synthesis apparatus 1 of the first embodiment. The image synthesis apparatus 1 is provided with an optical system 10 for image taking and a computation device 20. The optical system 10 has a first crystal (collimator) 11, which is an asymmetric Bragg case crystalline Si (440), and a second crystal (angular analyzer) 12, which is a symmetric Laue case crystalline Si (440), two X-ray cameras 13 and 14 disposed in directions in which X-rays travel, and a rotary stage 15 that rotates a subject Sa. The computation device 20 is connected to each of the X-ray cameras 13 and 14 and performs computations described below based on X-ray information obtained by the X-ray cameras 13 and 14 to synthesize an image of the subject Sa. The index of the crystal is not limited to (440) shown by way of example and may have any other suitable value.

The diffraction planes of the first crystal 11 and the second crystal 12 are parallel to each other. The subject Sa is disposed on the rotary stage 15 disposed between the first crystal 11 and the second crystal 12. In the present embodiment, the energy of incident X-rays (indicated by the arrow X in Figure 1) is assumed to be 34.8 keV. The X-rays are reflected by the first crystal 11 so that they are close to plane waves and the field of view is increased. The X-rays are then incident on the subject Sa on the rotary stage 15. Part of the X-rays are absorbed and refracted by the subject Sa. The X-rays are then led to the second crystal 12. At the second crystal 12, the X-rays are divided into forward diffracted waves A and diffracted waves B to be simultaneously detected with the CCD cameras 13 and 14, respectively.

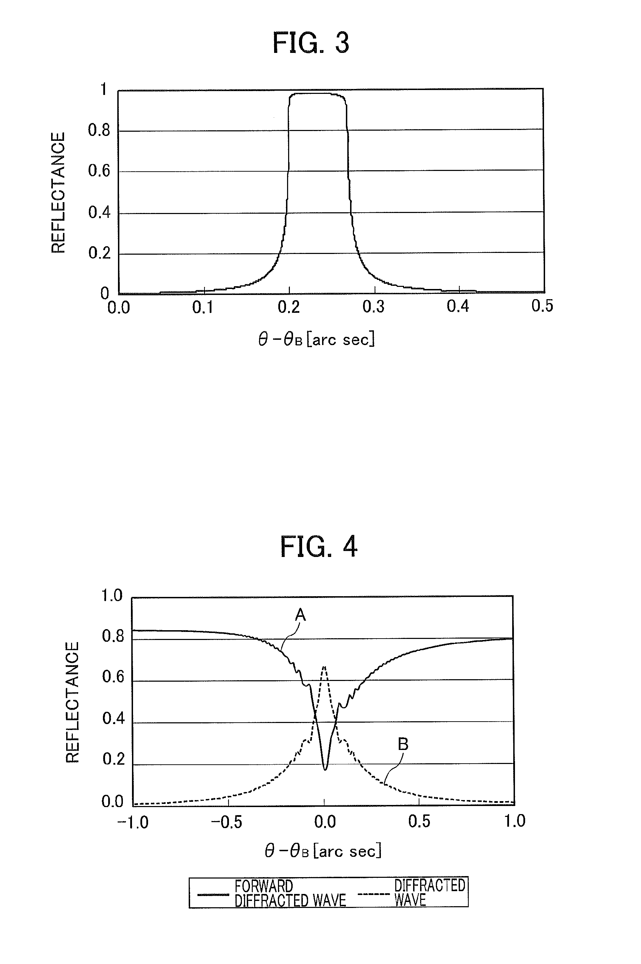

Figure 2 shows a theoretical forward diffraction curve A and a theoretical diffraction curve B with respect to the second crystal 12. These theoretical curves A and B are computed by assuming that there is no angular expansion of the beam incident on the second crystal 12. The X-rays reflected by the first crystal 11 are close to plane waves but expand slightly. Therefore the beam incident on the second crystal 12 has a slight angular expansion in actuality. Figure 3 shows by way of example a diffraction intensity curve of diffraction of X-rays at the Bragg case in a case where the expansion is up to on the order of 6/100 a second. Since the beam incident on the second crystal 12 has an angular expansion as described above, the forward diffraction curve A and the diffraction curve B with respect to the second crystal 12 are actually convolutions of the intensity curve (theoretical value) of the passed X-rays after reflection on the first crystal 11, shown in Figure 3, and the intensity curves (theoretical values) of the X-rays passed through the second crystal 12, shown in Figure 2. Figure 4 shows the results of obtaining the convolutions. It is desirable that the incident X-rays have a slight angular expansion because it is necessary that the intensity curve be smoothed by being cleared of vibration components when the refraction angle $\theta_0$ is obtained from the measured X-ray intensity.

(Principle of Image Taking)

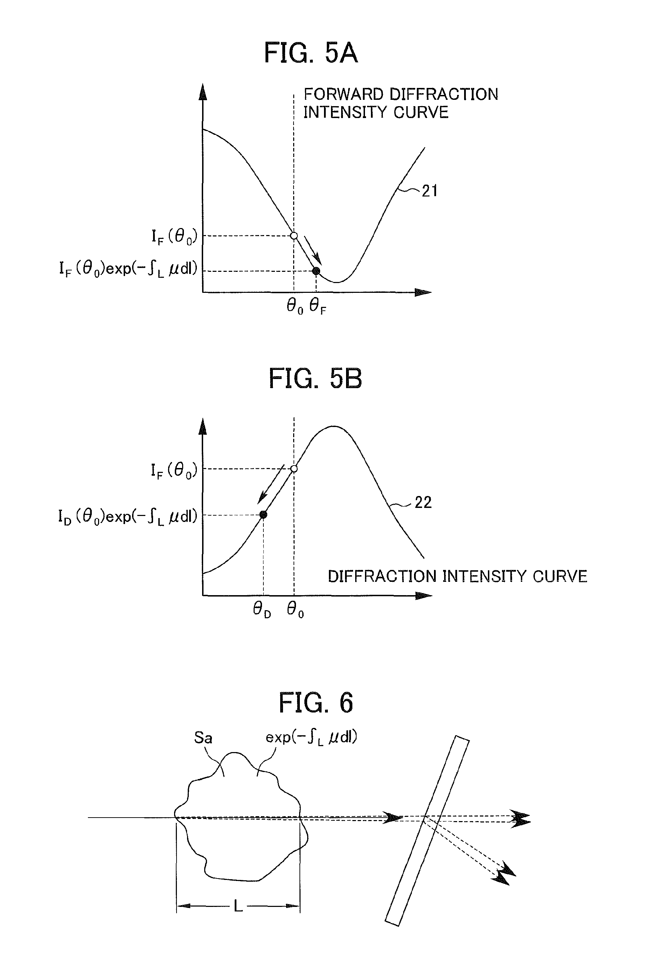

Figure 5A shows a forward diffraction intensity curve 21 representing the intensity characteristic of the forward diffracted waves A of the angular analyzer 12, and Figure 5B shows a diffraction intensity curve 22 representing the intensity characteristic of the diffracted waves B. In each of these figures, the abscissa represents the diffraction angle and the ordinate represents the intensity I of X-rays. The refraction angle $\theta_0$ of X-rays at the subject Sa can be estimated by applying the X-ray intensity I obtained through the CCD camera 13 to the forward diffraction intensity curve 21 or the diffraction intensity curve 22, if any intensity attenuation is not caused due to absorption in the subject Sa. The roles of the forward diffraction and the diffraction may be reversed.

However, as shown in Figure 6, when the incident X-rays pass through a route L in the subject Sa, its intensity attenuates at an attenuation rate $\exp(-\int_L \mu dl)$. Therefore the forward diffraction intensity $I_{Fdet}$ actually measured is $I_F(\theta_0)\exp(-\int_L \mu dl)$ and the diffraction intensity $I_{Ddet}$ is $I_D(\theta_0)\exp(-\int_L \mu dl)$, as shown in Figure 5. If diffraction angles in the intensity curves 21 and 22 are obtained from these actually measured intensities $I_{Fdet}$ and $I_{Ddet}$, erroneous refraction angles $\theta_F$ and $\theta_D$ are obtained, while the actual value is $\theta_0$.

Given the fact that the rate $\exp(-\int_L \mu dl)$ of attenuation in a subject is common to the forward diffraction and the diffraction, $\theta_0$ is estimated by solving simultaneous equations. In actuality, the intensity curves 21 and 22 are approximated with N-degree polynomials and solved by using the Newton's method. An example of approximation with quadratic polynomials will be described below. The intensity curves 21 and 22 are respectively expressed by the following equations.

$$I_F(\theta) = a_F\,\theta^2 + b_F\theta + c_F \qquad\qquad (1)$$

$$I_D(\theta) = a_D\,\theta^2 + b_D\theta + c_D \qquad\qquad (2)$$

$I_F(\theta)$ represents the forward diffraction intensity curve 21 and $I_D(\theta)$ represents the diffraction intensity curve 22. The actual refraction angle $\theta_0$ satisfies

$$I_F(\theta = \theta_0) = a_F\,\theta_0^{\,2} + b_F\theta_0 + c_F \qquad\qquad (3)$$

$$I_D(\theta = \theta_0) = a_D \theta_0^2 + b_D \theta_0 + c_D \qquad (4)$$

The relationships between the intensities $I_{Fdet}$ and $I_{Ddet}$ obtained by measurement and the true intensities $I_F(\theta_0)$ and $I_D(\theta_0)$ are given by the following equations if the attenuation rate $\exp(-\int_L \mu dl)$ is used.

$$I_F(\theta = \theta_0) = \frac{I_{F\,det}}{\exp(-\int_L \mu dl)} \qquad (5)$$

$$I_D(\theta = \theta_0) = \frac{I_{D\,det}}{\exp(-\int_L \mu dl)} \qquad (6)$$

From equations (3) to (6), the following equation is obtained.

$$\left(a_F - \frac{I_{F\,det}}{I_{D\,det}} a_D\right)\theta_0^2 + \left(b_F - \frac{I_{F\,det}}{I_{D\,det}} b_D\right)\theta_0 + \left(c_F - \frac{I_{F\,det}}{I_{D\,det}} c_D\right) = 0 \qquad (7)$$

By solving this equation, the actual refraction angle $\theta_0$ is obtained. In this example, since only the quadratic equation is solved, the analytic solution $\theta_0$ can be estimated.

If the degree of the approximating polynomials is increased, the accuracy of approximation is correspondingly increased. An N-degree polynomial corresponding to equation (1) is as shown below.

$$I_F(\theta) = \sum_{n=0}^{N} a_n \theta^n \qquad (1\,n)$$

Also, an N-degree polynomial corresponding to equation (7) is as shown below.

$$\sum_{n=0}^{N} \left(a_{Fn} - \frac{I_{F\,det}}{I_{D\,det}} a_{Dn}\right)\theta_0^n = 0 \qquad (7\,n)$$

Calculation shown below is performed by using $\theta_0$ thus obtained. The X-ray diffraction angle $\theta_0$ is calculated by the following equation as an integral over the X-ray beam path S by elemental refraction.

$$\theta_0 = \int_S |\nabla \tilde{n}(r)| \sin \varphi(r) dr \qquad (8)$$

where ñ(r) is a local refractive index real part in a relationship ñ=1-n with a refractive index n(r) at a position r (hereinafter referred to as "local refractive index real part" as occasion demands).

$\phi$(r) is the angle between the X-ray beam direction and a refractive index gradient $\nabla$ñ(r) i.e., the angle between the direction in which X-rays travel and the differential of the "local refractive index real part".

In this integration, if the "local refractive index real part" is $10^{-5}$ or less in the X-ray region, the X-ray path S can be approximated with a straight line.

Equation (8) is not adequate for CT resynthesis, because equation (8) has two unknown functions, i.e., the absolute value of a "refractive index real part gradient" and $\phi$(r), and because these cannot be obtained independently.

Since the values of the "local refractive index real part" at opposite sides of the object are equal to each other, the difference therebetween is always zero, as shown below.

$$0 = \int_{S} \left| \nabla \widetilde{n}(r) \right| \cos \varphi(r) dr \qquad (9)$$

If equation (9) is multiplied by the complex element i, and if the resulting product is added to equation (8), equations (8) and (9) can be expressed in complex form, as shown below.

$$\theta_0 = -i \int_{S} \left| \nabla \widetilde{n}(r) \right| e^{i\varphi(r)} dr \qquad (10)$$

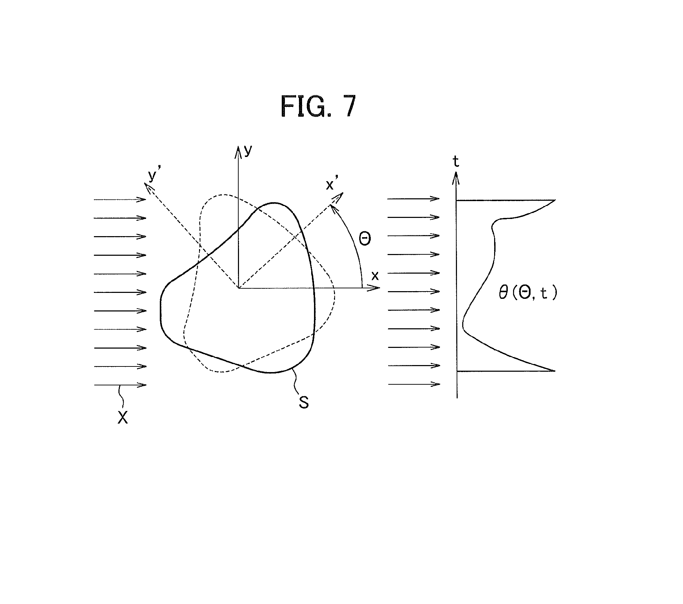

This equation is useful as a basis for CT resynthesis. Figure 7 shows a basic CT construction. The subject Sa is rotatably placed in X-rays. $\theta$ denotes the angle between the initial position and the present position (indicated by the broken line) of the object 12; t, a projection coordinate axis perpendicular to X-rays; and a refraction angle distribution $\theta_0$ ($\Theta$, t), a projection having information on the refraction angle.

According to an ordinary CT method, it is necessary to obtain information on the refraction angle as information on the angular position $\Theta$ and the spatial coordinate t of the object. Therefore an algorithm for CT resynthesis after mathematical processing by equation (10) can be expressed by the following equation.

$$\theta_0(\Theta, t) e^{i\Theta} = \int_{S} \left| \nabla \widetilde{n}(r) \right| e^{i\varphi(r)} dr \qquad (11)$$

The integration path S is a straight line xcos$\Theta$ + ysin$\Theta$= t.

Equation (11) shows that the results of CT resynthesis are gradients in a refractive index field. It will be noticed that this equation (11) is closely similar to an equation for CT resynthesis based on absorption contrast. The two equations differ in that the input function in the case of refraction contrast is $\theta_0$ ($\Theta$, t)exp(i$\Theta$) while the input function in the case of absorption contrast is log (I($\Theta$, t)/I$_0$), and that the function to be resynthesized in the case of refraction contrast is $\left| \nabla \widetilde{n}(r) \right|$exp($i\varphi$(r)) while the function to be resynthesized in the case of absorption contrast is $\mu$(r).

Further, there is an important difference between the cases of refraction contrast and absorption contrast. While the input function and the output function in the equation for CT synthesis based on absorption contrast are in the real-number space, CT synthesis based on refraction contrast uses the functions in the complex-number space. This means that the CT algorithm based on absorption contrast cannot be adopted in the case of refraction contrast, and that an original algorithm and software are required in the case of refraction contrast.

Because of the composition of equation (11), the mathematical form of the CT algorithm based on refraction contrast is the same as that of the algorithm (so-called Filtered Backprojection method) used in the case of absorption contrast. Basically, the mathematical form consists of four steps. That is,

i) Fourier transform of the input function $\theta_0$($\Theta$, t)exp(i$\Theta$) (called "sinogram" in many cases):

$$P_{\Theta}(\omega) = \int_{-\infty}^{\infty} \theta_0(\Theta, t) \exp(i\Theta) e^{-2\pi i \omega t} dt \qquad (1\,2)$$

ii) Filtering of the Fourier-transformed function $P_{\Theta}(\omega)$:

$$S_{\Theta}(\omega) = P_{\Theta}(\omega)\, b(\omega) \qquad (1\,3)$$

where $b(\omega)$ is a filtering function. There are many filtering functions used for CT resynthesis. However, filtering functions ordinarily used for CT based on absorption contrast are of almost no use in the case of refraction contrast. This is because all of these filtering functions intensify low-frequency components while suppressing high-frequency components. This is reasonable in the case of absorption contrast because most of useful information is contained in a low domain, and because most of high-frequency components are formed of noise. In contrast, in the case of refraction contrast, high-order Fourier components play an important role and are not suppressed by the filtering function.

iii) Backward Fourier transform of the filtered function:

$$Q_{\Theta}(t) = \int_{-\infty}^{\infty} S_{\Theta}(\omega) |\omega| e^{2\pi i t \omega} d\omega \qquad (1\,4)$$

The obtained function $Q_{\Theta}(t)$ is known as filtered sinogram. iv) Backprojection of the filtered sinogram into the real-number space:

$$|\nabla \widetilde{n}(r)| \exp(i\varphi(r)) = \int_0^{\pi} Q_{\Theta}(t) d\Theta \qquad (1\,5)$$

where $r \equiv (x, y)$ corresponds to t. $t = x\cos\Theta + y\sin\Theta$. This algorism is given for the continuous form of the equation. In all practical applications, however, the function $\theta_0(\Theta, t)$ is known only at particular points $\Theta_m$ and $t_n$. It is, therefore, necessary to use algorithms in individual forms for actual calculation.

CT resynthesis equation (11) based on refraction contrast shows the following. That is, the resynthesized function is the gradient of refractive index real part $\nabla \widetilde{n}(r)$. Most users would rather obtain the results in the form of the actual physical value $\widetilde{n}(r)$ than the gradient. To calculate the physical value, CT resynthesis is first executed. Then, by using the characteristic of the scalar field gradient:

$$\widetilde{n}(r_0) = \int_{\infty}^{r_0} \nabla \widetilde{n}(r) dr \qquad (1\,6)$$

from the gradient of refractive index real part $\nabla \widetilde{n}(r)$, the scalar field $\widetilde{n}(r)$ is formed.
However, the basic equation $\nabla \times (\nabla \widetilde{n}(r)) \equiv 0$ is not strictly satisfied because of a computation error in step (iv) of the CT resynthesis algorithm (see equation 15). This means that the value of the scalar field $\widetilde{n}(r)$ depends on selection of an integration path and, therefore, two or more integration equations (16) are calculated along various integration paths and the average of the integrals needs to be used as the most ideal result. To avoid this problem, another method for resynthesis of the value $\widetilde{n}$ was used. Gradient-field transform can be performed before the backprojection by the filtered projection equation (15), because the physical meaning of the filtered sinogram $Q_{\Theta}(t)$ is projection of the gradient $\widetilde{n}(r)$. In step (iv) of the resynthesis algorithm, therefore, a new function:

$$Q_\Theta^{\text{int}\,egrated}(t) = \int_\infty^t Q_\Theta(\tau)d\tau \qquad\qquad (1\ 7)$$

is used in place of the function $Q_\Theta(t)$. After this transform, the function resynthesized from equation (15) is not the gradient but the very refractive index. The gradient-field transform built in the resynthesis algorithm has certain advantages over the transform executed after resynthesis. Firstly, the integration equation (17) is one-dimensional in contrast to the integration equation (16) executed with respect to the curve on the surface (x, y). This mathematically facilitates the integration and makes the computation inexpensive.

Secondly, the equation $\nabla \times \nabla \tilde{n}(r) \equiv 0$ is, strictly effective before step (iv) of the algorithm by the term $|\omega|$ in the function to be integrated in equation (14). Equation (7) ensures that the average of the function $Q_\Theta(t)$ is equal to zero.

(Example 1: Verification of effectiveness by simulation)

The effectiveness was verified by using above-described equations and by using a simulation in the DFI method. In this simulation, an approximation was made by using 6-degree polynomials in calculation of $\theta_o$. At this time, since no analytic solution can be obtained, the Newton's method was used for solution. In this computation, solutions in the case of approximation of the vicinities of $I_{Fdet}$ and $I_{Ddet}$ with linear equations were used as initial values.

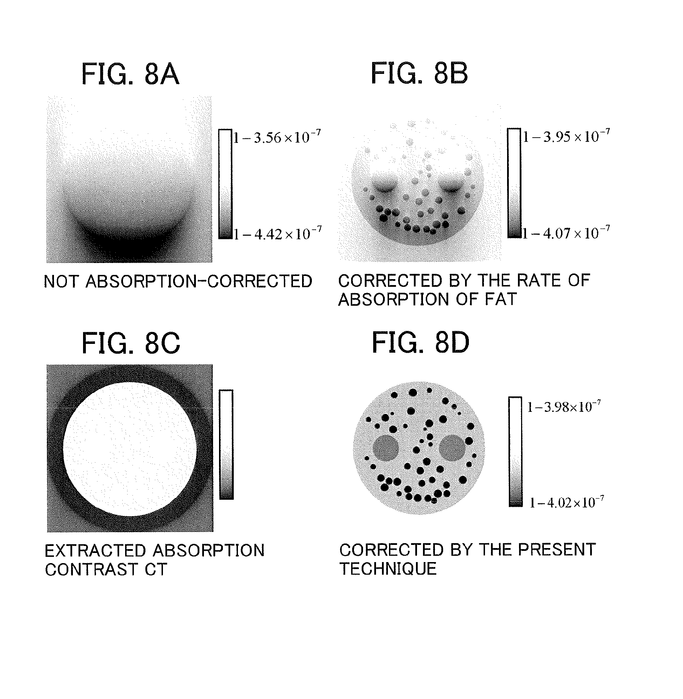

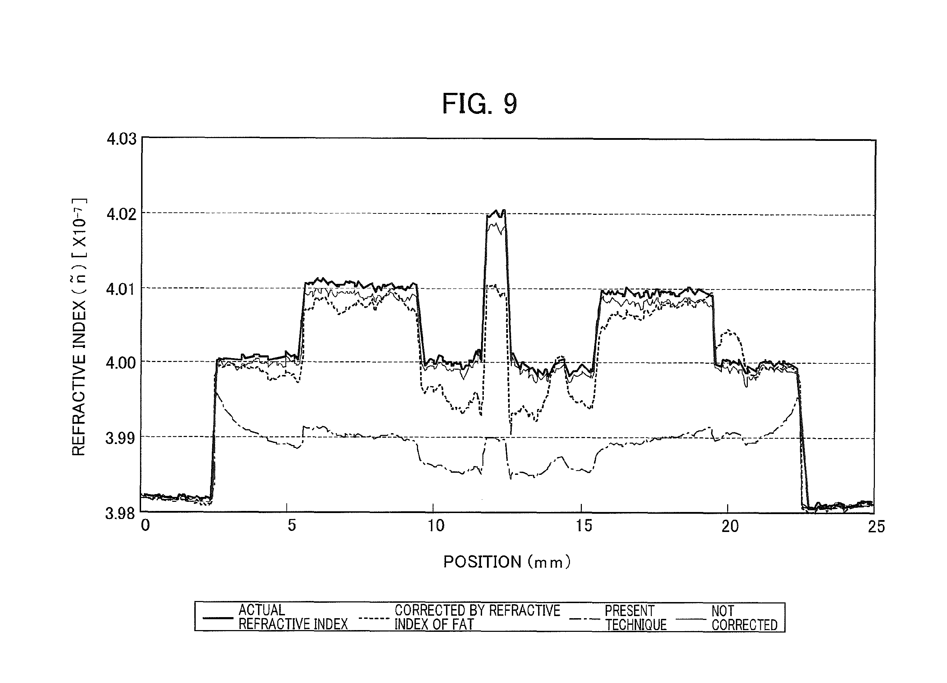

To evaluate the effectiveness of this technique, a simulator based on geometric optics with respect to X-rays (incident energy: 34.8 keV) was developed and an image taking experiment was performed. As a specimen, two cylinders having a diameter of 4 mm and small balls randomly disposed around the two cylinders were disposed in a cylinder having a diameter of 20 mm. Complex refractive indices of water, fat and a mammary gland were assumed. Figure 8A-8D show results of a CT reconstruction from projected images obtained by simulation: a non-absorption-corrected state, a state of being corrected with the absorption coefficient of fat by assuming that the entire specimen was fat, and a state of being corrected according to the present proposition. In the case of making no correction, substantially no contrast is produced for the internal cylinders and the balls. Making a correction with the absorption coefficient of fat is unsatisfactory in correction about the circles but is generally effective in improving the contrast. This is because the absorption coefficients of water, fat and the mammary gland are close to each other. By the correction according to the present proposition, a generally perfect refraction CT image can be obtained. Figure 9 is a graph of the refractive index real part n(r) (=1-n), where n is the refractive index, for one line at a position of 12. 5 mm in the vertical direction in each CT image. Also in quantitative effect, the refractive index obtained by the present technique is close to the true refractive index.

(Example 2: a three-dimensional image of a human mammary cancer tissue)

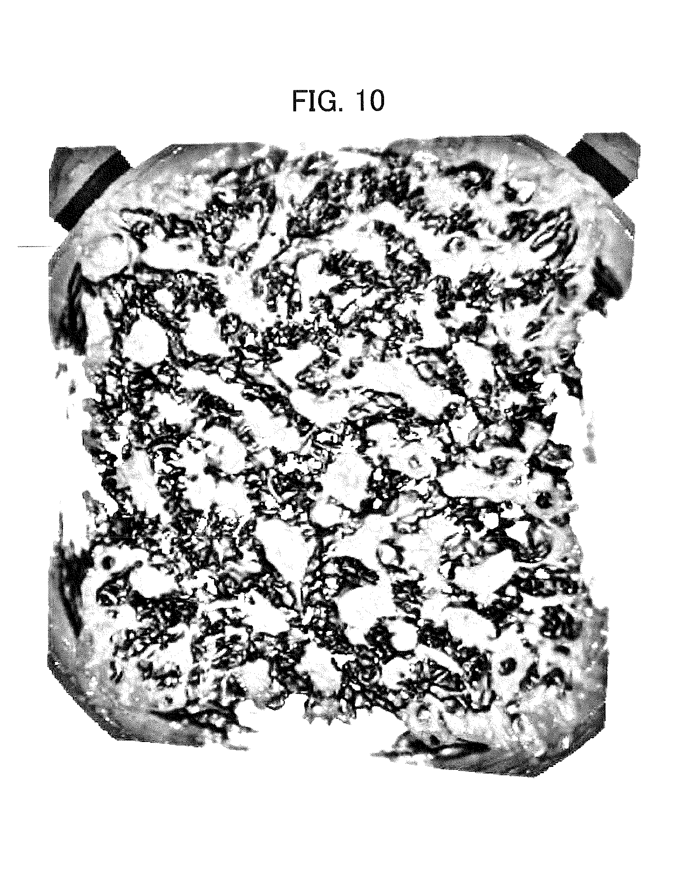

Figure 10 is a photograph actually taken from a prepared subject Sa by using the technique according to the present embodiment.

The subject Sa is a human DCIS mammary cancer borrowed from National Hospital Organization Nagoya Medical Center. This subject Sa has a size of a 25 mm square and a thickness of 33 mm and is immersed in a 5% formalin solution. This subject Sa was inserted in a Teflon (trademark) cylinder having a diameter of 35 mm for the purpose of enabling the subject Sa to be easily handled without being displaced, for example, when rotated at the time of image taking. The thickness of Teflon (trademark) is 0.05 mm. The gap between the specimen and the cylinder was filled with agarose. To fill this gap, alcohol and agar may alternatively be used. The subject Sa inserted in this cylinder was placed in the position shown in Figure 1 on a goniometer, a product from Kohzu Precision Co., Ltd, provided as the rotary stage 15. Image taking from the subject Sa was performed at a rate of 900 shots/per 0.2 degree by rotating the goniometer 15. X-rays were applied at 35 keV and $10^8$ photons/mm²/second. As the CCD camera, "X-Ray VHR2, 60 mm", a product from Photonic Science Limited, UK, was used. For image data obtained by the present method, VGStudioMAX, a product from Volume Graphics GmbH, was used as the computation device 20.

According to the present embodiment, as shown in the figure, a high-contrast subject photograph was obtained.

(Second Embodiment)

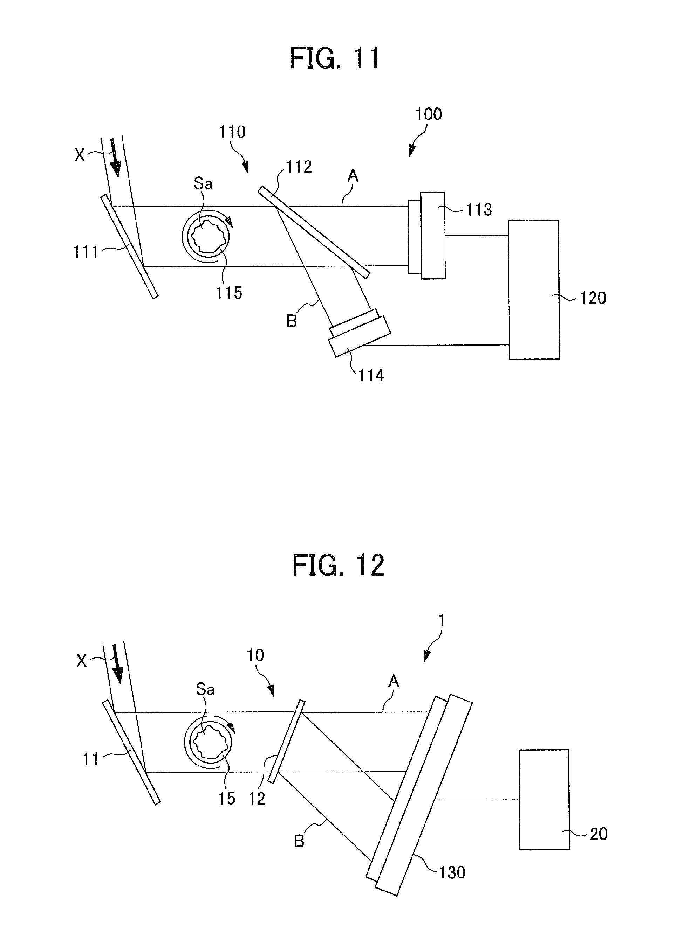

A second embodiment of the present invention using an X-ray Diffraction-Enhanced Imaging (DEI) method will be described below. Figure 11 is a diagram schematically showing an image synthesis apparatus in the second embodiment. The first embodiment and the second embodiment differ from each other in that a second crystal (angular analyzer) 112 is a reflection-type angular analyzer.

In the second embodiment, X-rays are incident on the subject Sa on a rotary stage 115, absorbed and refracted by the subject Sa, and led to the second crystal 112. At the second crystal 112, the X-rays are divided into forward diffracted waves A and reflected diffracted waves B to be simultaneously detected with the CCD cameras 113 and 114, respectively. The way of obtaining the diffraction angle $\theta_0$ and the image synthesis method using the diffraction angle $\theta_0$ are the same as those in the first embodiment.

(Third Embodiment)

A third embodiment of the present invention using an X-ray Dark-Field Imaging (DFI) method will be described below. Figure 12 is a diagram schematically showing an image synthesis apparatus in the third embodiment. The first embodiment and the third embodiment differ from each other in that only one CCD camera 130 is provided, that is, the forward diffraction intensity curve and the diffraction intensity curve are simultaneously detected with one CCD camera 130. The way of obtaining the diffraction angle $\theta_0$ and the image synthesis method using the diffraction angle $\theta_0$ are the same as those in the first embodiment. Use of one CCD camera 130 in this way enables the entire image synthesis apparatus 1 to be constructed at a lower cost.

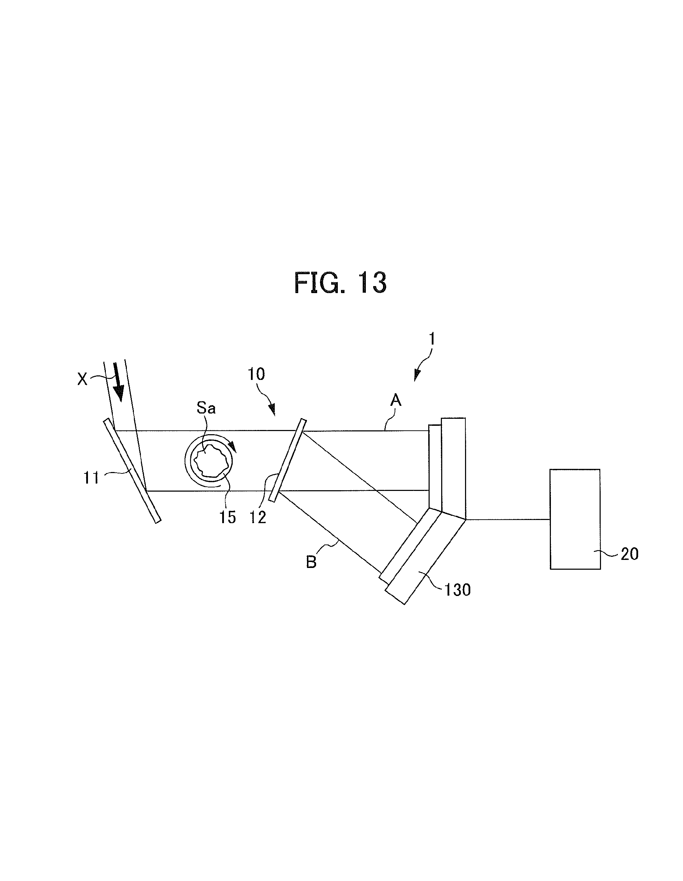

Figure 13 shows a modified example of the third embodiment in which only one CCD camera 130 provided can be bent at its center and the angle between the bent portions can be adjusted. In this modified example, each of the forward diffracted wave A and the reflected diffracted wave B is perpendicularly incident on the CCD camera 130, thereby facilitating computation processing thereafter performed.

The advantages of the present embodiments will be summarized below.

(1) From a soft part tissue, which is not discriminable by means of absorption contrast, an image can be taken with high contrast by CT image taking using diffracted X-rays.
(2) Absorption information and diffraction information can be obtained separately from each other when rays are applied once per measurement point. Therefore two CT images by absorption and diffraction can be obtained and a quantitative diagnosis can be made on each image.
(3) Because of image taking by applying rays one time, the exposure can be reduced to 1/2 or less in comparison with the DEI method.
(4) Because data is obtained by using an analyzer, high spatial resolution can be achieved without the influence of scattered rays.

**Claims**

**1.** An image synthesis apparatus comprising:

an angular analyzer that separates a parallel beam applied to a subject into a forward diffracted beam and a diffracted beam;
a detection device that detects a first forward diffracted beam intensity, which is the intensity of the forward diffracted beam separated by the angular analyzer, and a first diffracted beam intensity, which is the intensity of the diffracted beam; and
a computation device that obtains a refraction angle of the beams by the subject from a forward diffraction curve representing the relationship between the intensity of the forward diffracted beam and the refraction angle and a diffraction curve representing the relationship between the intensity of the diffracted beam and the refraction angle, the curves representing characteristics of the angular analyzer,
by assuming that an attenuation rate at the first forward diffracted beam intensity and an attenuation rate at the first diffracted beam intensity at the time of passage through the subject are equal to each other, and
by using a second forward diffracted beam intensity and a second diffracted beam intensity from which the influence of attenuation is removed, the second forward diffracted beam intensity and the second diffracted beam intensity being obtained by correcting the first forward diffracted beam intensity and the first diffracted beam intensity by the attenuation rate,
the computation device synthesizing an image of the subject from the refraction angle.

**2.** The image synthesis apparatus according to claim 1, wherein the computation device approximates the forward diffraction curve with an n-degree polynomial to obtain a new forward diffraction curve, approximates the diffraction curve with an n-degree polynomial to obtain a new diffraction curve, thereafter if it is assumed that the X-rays that pass through the subject in the forward diffraction and the diffraction receive equal amounts of attenuation from the subject, an n-degree equation to be satisfied by the corresponding two observed intensities can be derives. By solving the equation, , the refraction angle can be obtained.

3. The image synthesis apparatus according to claim 1 or 2, wherein the angular analyzer is a transition-type angular analyzer or a reflection-type angular analyzer.

4. The image synthesis apparatus according to any one of claims 1 to 3, wherein the beam is electromagnetic waves or neutron rays.

5. The image synthesis apparatus according to any one of claims 1 to 4, wherein the detection device has a first detection device that detects the forward diffracted beam separated by the angular analyzer, and a second detection device that detects the diffracted beam.

6. An image synthesis method comprising:

   separating a parallel beam applied to a subject into a forward diffracted beam and a diffracted beam by means of an angular analyzer;
   detecting a first forward diffracted beam intensity, which is the intensity of the forward diffracted beam separated by means of the angular analyzer, and a first diffracted beam intensity, which is the intensity of the diffracted beam; and
   obtaining a refraction angle of the beams by the subject from a forward diffraction curve representing the relationship between the intensity of the forward diffracted beam and the refraction angle and a diffraction curve representing the relationship between the intensity of the diffracted beam and the refraction angle, the curves representing characteristics of the angular analyzer,
   by assuming that an attenuation rate at the first forward diffracted beam intensity and an attenuation rate at the first diffracted beam intensity at the time of passage through the subject are equal to each other, and
   by using a second forward diffracted beam intensity and a second diffracted beam intensity from which the influence of attenuation is removed, the second forward diffracted beam intensity and the second diffracted beam intensity being obtained by correcting the first forward diffracted beam intensity and the first diffracted beam intensity by the attenuation rate, and
   synthesizing an image of the subject from the refraction angle.

# FIG. 1

# FIG. 2

## FIG. 3

## FIG. 4

# FIG. 5A

FORWARD DIFFRACTION INTENSITY CURVE

# FIG. 5B

DIFFRACTION INTENSITY CURVE

# FIG. 6

# FIG. 7

## FIG. 8A

$1 - 3.56 \times 10^{-7}$

$1 - 4.42 \times 10^{-7}$

NOT ABSORPTION-CORRECTED

## FIG. 8B

$1 - 3.95 \times 10^{-7}$

$1 - 4.07 \times 10^{-7}$

CORRECTED BY THE RATE OF
ABSORPTION OF FAT

## FIG. 8C

EXTRACTED ABSORPTION
CONTRAST CT

## FIG. 8D

$1 - 3.98 \times 10^{-7}$

$1 - 4.02 \times 10^{-7}$

CORRECTED BY THE PRESENT
TECHNIQUE

# FIG. 9

| ACTUAL REFRACTIVE INDEX | CORRECTED BY REFRACTIVE INDEX OF FAT | PRESENT TECHNIQUE | NOT CORRECTED |

# FIG. 10

## FIG. 11

## FIG. 12

# FIG. 13

| INTERNATIONAL SEARCH REPORT | | International application No. |
|---|---|---|
| | | PCT/JP2010/069350 |

**A. CLASSIFICATION OF SUBJECT MATTER**
*G01N23/04*(2006.01)i, *A61B6/03*(2006.01)i

According to International Patent Classification (IPC) or to both national classification and IPC

**B. FIELDS SEARCHED**

Minimum documentation searched (classification system followed by classification symbols)
G01N23/00-23/227, A61B6/00-6/14

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 2001-33406 A (NEC Corp.), 09 February 2001 (09.02.2001), paragraphs [0028] to [0035]; fig. 1, 2 (Family: none) | 1-6 |
| A | JP 2009-8449 A (Hitachi, Ltd.), 15 January 2009 (15.01.2009), paragraphs [0025] to [0045]; fig. 12 & US 2009/0003517 A1 & EP 2009429 A1 | 1-6 |
| A | JP 2003-329617 A (Masami ANDO), 19 November 2003 (19.11.2003), paragraph [0018]; fig. 1, 5 & US 2004/0196957 A1 & US 2008/0298551 A1 & EP 1429138 A1 & WO 2003/008952 A & WO 2003/008952 A1 | 1-6 |

| ☐ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 November, 2010 (26.11.10) | 07 December, 2010 (07.12.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006090925 A